# EUROPEAN PATENT SPECIFICATION

(11) **EP 2 066 264 B1**
(45) Date of publication and mention of the grant of the patent: **12.01.2011**
(21) Application number: 07842094.0
(22) Date of filing: 07.09.2007
(51) Int. Cl.: A61F 2/16

(54) **ENHANCEMENT OF LENS REGENERATION USING MATERIALS COMPRISING POLYMERS**
VERBESSERTE LINSENREGENERATION DURCH VERWENDUNG VON MATERIALIEN MIT POLYMEREN
AMÉLIORATION DE LA RÉGÉNÉRATION DU CRISTALLIN PAR L'UTILISATION DE MATÉRIAUX COMPRENANT DES POLYMÈRES

(30) Priority: 07.09.2006 US 470724
(43) Date of publication of application: 10.06.2009
(73) Proprietor: Abbott Medical Optics Inc., Santa Ana, CA 92705-4933 (US)
(72) Inventor: GWON, Arlene, Newport Beach, CA 92660 (US)
(74) Representative: HOFFMANN EITLE
(86) International application number: PCT/US2007/077930
(87) International publication number: WO 2008/031066

(56) References cited:
- WO-A-2006/069012
- US-A1- 2006 002 981

## Description

### FIELD OF THE INVENTION

The present invention addresses the treatment of ocular conditions by the enhancement of lens regeneration. Enhancement of lens regeneration is accomplished through the administration of a composition comprising a polymer having functional groups that is useful in the preparation of intraocular lenses (IOLs).

### BACKGROUND OF THE INVENTION

A cataract is the clouding of a natural eye lens, the part of the eye that focuses light onto the retina to produce clear, sharp images. The lens is contained in a sealed bag or capsule. As old lens cells die they become trapped within the capsule and, over time, the accumulation of these cells causes the lens to cloud, so that light is no longer focused properly onto the retina and images appear blurred or fuzzy. For most people, cataracts are a natural result of aging.

A process called extracapsular cataract extraction with implantation of an intraocular lens (IOL) is currently the most common method for the treatment of cataracts. This process involves removing the natural dysfunctional lens and replacing it with an artificial lens. This procedure is less than ideal, however, because the current synthetic IOLs are unable to accommodate appreciably, and secondary opacification of the posterior capsule (i.e. secondary cataracts) is a common occurrence following the procedure.

Importantly, after removal, in some situations eye lenses can regenerate over time. Ideally, if a regenerated natural lens could replace a suitable biodegradable material, the reformed regenerated lens could have the same or similar natural focusing power as the normal young lens and could be able to accommodate visually. Alternatively, if naturally regenerating lens epithelial cells could be directed to grow in a regularly organized pattern around a suitably flexible and biocompatible polymeric lens, the resultant bilenticular system might also be able to accommodate. Therefore, there is a need in the art for a regenerated lens (with or without a suitably flexible and biocompatible polymeric lens) which would have properties of the natural lens including clarity, protein content, histology, focusing power, spectral transmission, accommodative ability, configuration, shape and structure.

One approach to forming a lens following cataract extraction has been to use accommodative refill lenses. Accommodative refill lenses are created by injecting liquids (such as silicone oils or low temperature vulcanizing (LTV) silicone elastomers) into the len's capsular bag through a small incision. After injection, these liquids polymerize under forming pressure to create a lens of the required shape. This technique uses the form of the capsular bag as a mold. Various drawbacks associated with this technique remain to date, however, including scarring and folds in the capsule, epithelial cell proliferation and secondary capsular opacification, preventing its beneficial use in all patients. The present invention provides beneficial methods of using new materials to create accommodative refill lenses and to enhance natural lens regeneration.

### DEFINITION OF TERMS

Accommodation: As used herein, "accommodation" refers to the eye's ability to automatically change focus from seeing at one distance to seeing at another.

Enhance lens regeneration: As used herein, "enhance lens regeneration" and variants of the same phrase include increasing the probability of a lens regenerating after extracapsular lens extraction wherein the lens will have less haze, less striae or fewer folds than it otherwise would have had without a described treatment or is a regenerative lens that is more clear than it otherwise would have been with a described treatment.

Functional acryl groups: As used herein, "functional acryl groups" include molecules having functional groups attached thereto including an acryl group moiety, so as to become acryl-bearing, by acryl attachment to the monomers of a backbone, its terminal ends, or both. The acryl groups in these functional groups can be linked to the atoms by spacers. Examples of functional acryl groups include, without limitation, acrylamidopropyl, methacrylamidopropyl, acryloxyhexyl and methacryloxyhexyl. In certain embodiments, "functional acryl groups" include polysiloxane molecules having functional groups attached thereto including an acryl group moiety, so as to become acryl-bearing, by acryl attachment to the siloxane monomers of the polysiloxane backbone, its terminal ends, or both. The acryl groups in these functional groups can be linked to the silicone atoms by spacers. In one embodiment, the functional acryl groups are attached to the terminal ends of polysiloxane molecules, as exemplified by, without limitation, acrylamidopropyl-, methacrylamidopropyl-, acryloxyhexyl- and methacryloxyhexyl-terminated polysiloxanes. Those skilled in the art can consider numerous such alternatives which maintain the basic function of having an acryl group for subsequent crosslinking/polymerization of the polysiloxane molecules into larger network together with a photoinitiator. In the same manner it is also to be understood that the meaning of acryl group includes acryl or substituted acryl, such as, without limitation, methacryl, moieties attached through a variety of linkages including ester, amide and urethane linkages, or functional analogues of acryl capable of undergoing crosslinking reactions with a photoinitiator.

### DETAILED DESCRIPTION

The present invention is based on the concept that the natural lens is capable of controlled or enhanced organic cellular or biological regeneration following endocapsular lens and/or cataract extraction. In various embodiments, the present invention according to claim 1 provides an injectable lens material to contribute to the production of a regenerated lens with properties similar to that of the natural lens, including, without limitation, clarity, protein content, histology, focusing power, spectral transmission and accommodative ability. In one embodiment, the natural regenerating lens tissue can be directed to grow in a more natural or regular pattern around a suitably flexible and biocompatible polymeric lens.

The eye's regenerative lens fibers can be generated in the eye's cortex. These regenerated lens fibers originate as epithelial cells and elongate into ribbon-like nucleus-free lens components. The cross sections of these lens components are hexagonal in shape.

The concept of implanting a suitably flexible polymeric lens compatible with naturally regenerating lens tissue was previously suggested by studies in which Acuvue^{®} contact lenses (etafilcon A, 58% H₂O; available from Johnson & Johnson Vision Care, Inc., Jacksonville, Florida) were modified for intralenticular implantation in the rabbit eye. While normal regeneration was noted in one eye, the results were inconsistent and the nucleus of most regenerated lenses contained a star-shaped opacity related to the irregular growth pattern and misalignment of the earliest lens fibers.

In numerous studies, lenses that have been regenerated following endocapsular lens extraction in New Zealand Albino rabbits have been irregular in shape, appearing primarily doughnut-shaped. The newly formed lenses are irregular in shape as a result of the lack of lens growth at the site of the anterior capsulotomy and its adhesion to the posterior capsule. These regenerated lenses have had variable translucency because of irregular alignment of newly formed fibers, which may partly result from irregular proliferation of cells in zones of wrinkling or folding of the lens capsule in the early postoperative period. To improve the transparency of the regenerated lenses and their therapeutic utility, investigators have attempted to mimic the embryonic environment with limited success.

The various embodiments of the present invention reaffirm past findings in the art showing lens regeneration and demonstrate that regeneration of the lens can be enhanced through the use of polysiloxane polymer containing compositions. Certain embodiments according to the present invention also incorporate the use of viscoelastic materials including, without limitation, hyaluronic acid, cellulosic materials, collagen, and combinations thereof to further enhance lens regeneration. Embodiments according to the present invention can also enhance lens regeneration by restoring lens capsule integrity following the administration of polysiloxane polymer containing compositions.

The present invention provides methods of enhancing lens regeneration using materials comprising polymers. Certain embodiments include methods of enhancing lens regeneration using materials comprising polysiloxane polymers. Surgeons or researchers can inject the material into a capsular bag to form a lens or scaffold onto and around which lens cells can regenerate and organize.

Specifically, in one embodiment according to the present invention the invention is a method comprising enhancing regeneration of lens cells in a mammal after endocapsular extraction by filling a lens capsule bag of the mammal with an injectable lens material comprising a polymer wherein the injectable lens material has a viscosity for being injected through standard cannula and the polymer has functional groups at terminal ends of the polymer. In certain embodiments the polymer used in accordance with the present invention comprises a polysiloxane polymer. In certain other embodiments, the polymer used in accordance with the present invention comprises a polysiloxanes polymer with functional acryl groups.

In another embodiment of the methods, the endocapsular extraction occurs through a capsulorrhexis that is 3 millimeters or less.

Embodiment of the presently described methods can also further comprise one or more of: (i) inserting a foldable intraocular lens into the capsule bag of the mammal; (ii) positioning a contact lens or similar polymeric material in the form of a permeable or semi-permeable disc shaped lens material between the anterior capsule and the injectable lens material or between the injectable lens material and the posterior capsule; (iii) administering viscoelastic materials including, without limitation, one or more of hyaluronic acid, cellulosic materials, collagen, and combinations thereof (in one embodiment Healon^{®} brand hyaluronic acid) to the capsule bag of the mammal; (iv) administering hyaluronidase to the capsule bag of the mammal; and (v) inserting at least one collagen patch in the capsule bag of the mammal.

In another embodiment of the methods, the injectable lens material further comprises a photoinitiator and is capable of being photopolymerized into a solid intraocular lens.

In yet another embodiment of the methods, a polysiloxane polymer used in accordance with the present invention has a backbone of the general formula: 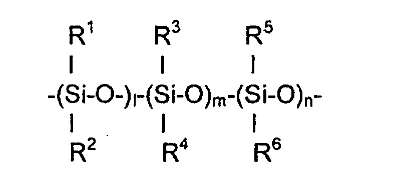 wherein R¹ and R² are independently C₁-C₆ alkyl; R³ is phenyl; R⁴ is phenyl or C₁ - C₆ alkyl; R⁵ is CF₃ (CH₂)ₓ wherein x is 1-5; R⁶ is C₁-C₆alkyl or fluoroalkyl; I is in the molar fraction range of 0 to 0.95; m is in the molar fraction range of from greater than 0 to 0.7; and n is in the molar fraction range of from greater than 0 to 0.65. As will be understood by one of ordinary skill in the art, in certain embodiments it will be beneficial to bind, x-link or otherwise join a viscoelastic material such as, without limitation, hyaluronic acid with this polysiloxane material (i.e. a fixed hyaluronic acid coating). This would facilitate the viscoelastic material staying positioned correctly.

The present invention also includes kits. Kits according to the present invention can comprises instructional materials and one or both of (i) a polysiloxane polymer; and (ii) a photocurable polysiloxane polymer and wherein the kit can also comprise one or more of (i) viscoelastic materials including, without limitation, one or more of hyaluronic acid, cellulosic materials, collagen, and combinations thereof; (ii) hyaluronidase; (iii) a collagen patch; (iv) an intraocular lens; and (v) a contact lens or similar polymeric material in the form of a permeable or semi-permeable disc shaped lens material. The instructional materials of these kits instruct the use of the included components in enhancing the regeneration of lens cells. In alternative embodiments, the instructional materials can also direct that the endocapsular extraction occurs through a capsulorrhexis that is 3 millimeters or less in diameter.

In another embodiment of the kits, the polysiloxane polymer has a backbone of the general formula: 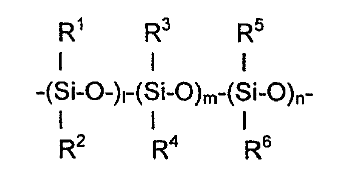 wherein R¹ and R² are independently C₁ -C₆ alkyl; R³ is phenyl; R⁴ is phenyl or C₁ - C₆ alkyl; R⁵ is CF₃ (CH₂)ₓ wherein x is 1-5; R⁶ is C₁ -C₆ alkyl or fluoroalkyl; I is in the molar fraction range of 0 to 0.95; m is in the molar fraction range of from greater than 0 to 0.7; and n is in the molar fraction range of from greater than 0 to 0.65. Again, and as will be understood by one of ordinary skill in the art, in certain embodiments it will be beneficial to bind, x-link or otherwise join a viscoelastic material such as, without limitation, hyaluronic acid with this polysiloxane material (i.e. a fixed hyaluronic acid coating).

The following *in vivo* studies evaluated the use of polymers, including polysiloxane polymers, following endocapsular lens extraction in young and old New Zealand white rabbits.

### Example 1

### General Materials

The polymer material used in the presently described studies included a polysiloxane polymer obtained from AMO Groningen B.V. (Groningen, Holland) that corresponded to composition AS4-11, CS0402014. Silicone plugs (4.5 mm) were also obtained from AMO Groningen B.V. (Groningen, Holland). "Shark Tooth" Phaco needles, (LAMINAR^{®} Flow Phaco Tip15°/45°) and infusion sleeve (20 gauge, OP0154520L) were obtained from Advanced Medical Optics, Inc. (Santa Ana, CA). Healon 5^{®} brand hyaluronic acid was obtained from AMO USA, Inc. (Santa Ana, California).

### General Methods

The general health and acceptability of animals used in the following studies was established prior to surgery. A total of 7 New Zealand white rabbits were used in three studies. During surgery, rabbits were anesthetized with about 5 mg/kg xylazine and about 50 mg/kg ketamine HCl, intramuscularly. The surgical eye was dilated with 1% cyclopentolate and 10% phenylephrine; eyelashes were trimmed; and the ocular area was disinfected with povidone iodine. A wire lid speculum was inserted to retract the lids, and a corneal incision was made at 12:00 with a 2.85 mm keratome. Healon 5^{®} brand hyaluronic acid was injected to maintain anterior chamber depth and an about 2-3 mm continuous curvilinear capsulorrhexis was performed. A 20 gauge phacoemulsification tip was inserted through the corneal wound and endocapsular lens extraction was performed by phacoemulsification and irrigation/aspiration with balanced salt solution (BSS). Considerable care was taken to remove all lens cortical material by diligent irrigation and aspiration. A 4.5 mm silicone plug was inserted into the capsule bag and maneuvered behind the anterior capsulotomy and the polysiloxane polymer material was injected into the capsule bag using a 20 gauge cannula. At the completion of the procedure, the corneal incision was closed with 10-0 nylon sutures and 0.25 ml (20 mg) of gentamicin and. 0.1 ml of dexamethasone (2mg) was injected subconjunctivally every 3 days for two weeks.

The overall positional stability of the silicone plug was evaluated based on the slit lamp observation of placement in the capsule bag. Observations of conjunctiva, corneal pathology, anterior chamber cells, flare and fibrin, posterior synechiae, capsular bag shape, and percent lens regrowth in the capsule bag were recorded.

All slit lamp findings were graded on a scale of 0 to 4 (0=none, 1+ = trace, 2+ = mild, 3+ = moderate, 4+ = severe). Throughout the experimental period, rabbits were observed for any abnormal clinical signs, including any abnormal ocular findings such as pain, excessive hyperemia or discharge. Grading of the ocular findings was based on the methods of McDonald and Shadduck (McDonald, T. O., and Shadduck, J. A. Eye Irritation. Advances in Modern Toxicology, Vol. 4, pp. 162-166. Dermatotoxicology and Pharmacology. Eds. Marzulli and Maibach. Washington: Hemisphere, 1977).
All observations were recorded on a Slit Lamp Examination form. Grading for each of these parameters was done as follows:

**Cornea:** Scores recorded for the cornea reflect the greatest severity of corneal edema/cloudiness observed. Severity of corneal cloudiness was graded as follows:

| | | |
|---|---|---|
| None | 0 | Transparent, clear |
| Trace | +1 | Minimal loss of transparency. Only the epithelium and/or the anterior half of the stroma is involved as observed with an optical section of the slit lamp. |
| Mild | +2 | Dull-glass appearance. The cloudiness extends past the anterior half of the stroma. |
| Moderate | +3 | Involvement of the entire thickness of the stroma. The affected stroma has lost its marble-like appearance and is homogeneously white. With optical section, the endothelium is still visible. |
| Severe | +4 | Involvement of the entire thickness of the stroma. With optical section, cannot clearly visualize the endothelium. |

**Cells in the anterior chamber:** The presence/absence of cells in the anterior chamber was monitored as follows:

| | | |
|---|---|---|
| None | 0 | No cells seen |
| Trace | +1 | 1-9 cells per high power field |
| Mild | +2 | Sparse and scattered or localized cells |
| Moderate | +3 | Numerous and scattered and/or clumped cells |
| Severe | +4 | High concentration of cells throughout most or the entire anterior chamber, and/or clumped and cascading down the anterior lens surface |

**Anterior chamber flare:** Anterior chamber flare was graded on the intensity of the Tyndall phenomenon and was scored by comparing the normal Tyndall effect observed when the slit lamp passes through the lens with that seen in the anterior chamber:

| | | |
|---|---|---|
| None | 0 | Absence of visible light beam in the anterior chamber (no Tyndall effect). |
| Trace | +1 | Tyndall beam is barely discernable. The intensity of the light in the anterior chamber is less than the intensity of the light beam as it passes through the lens. |
| Mild | +2 | The Tyndall beam in the anterior chamber is easily discernible and is barely equal in its intensity to the slit beam as it passes through the lens. |
| Moderate | +3 | The Tyndall beam in the anterior chamber is easily discernible and is equal in its intensity to the slit beam as it passes through the lens. |
| Severe | +4 | The Tyndall beam in the anterior chamber is easily discernible and its intensity is greater than the intensity to the slit beam as it passes through the lens. |

**Fibrin- in the anterior chamber:** The presence/absence of fibrin in the anterior chamber was monitored as follows:

| | | |
|---|---|---|
| None | 0 | No fibrin seen. |
| Trace | +1 | Minute strands or clumps of fibrin. |
| Mild | +2 | Thin sheet or clumps of fibrin. |
| Moderate | +3 | 20-50% of anterior chamber filled with fibrin. |
| Severe | +4 | Over 50% of anterior chamber filled with fibrin. |

**Posterior Synechia:** The presence/absence of iris adhesion to the lens capsule is known as posterior synechia and was graded as follows:

| | | |
|---|---|---|
| None | 0° | No posterior synechia seen. |
| Trace | 90° | Less or up to 90° of the iris pupil is scarred to the lens capsule (25% of pupil). |
| Mild | 180° | 180° of the iris pupil is scarred to the lens capsule (50% of pupil). |
| Moderate | 270° | 270°of the iris pupil is scarred to the lens capsule (75% of pupil). |
| Severe | 360° | 360°of the iris pupil is scarred to the lens capsule (100% of pupil). |

At the end of the studies, animals were euthanized by an injection of sodium pentobarbital (Eutha-6, Western Medical Supply Co., Inc.) into the marginal ear vein. At all times animals were treated in accordance with USDA guidelines and the ARVO Resolution on the Use of Animals in Research.

The study described in Example 1 evaluated: (i) posterior capsule opacification when a foldable silicone IOL in combination with the polysiloxane polymer material is used and (ii) the surgical technique for performing endocapsular extraction through a small 2 mm capsulorrhexis in an older rabbit with a hard lens.

### Example 1a

In this study, rabbit 71719 (New Zealand white female; weight 2.4kg) was 3 months old and rabbits 71565 and 71568 (New Zealand white females; weight 3.5 kg) were 3 years old.

Rabbit 71719 OD, OS: Uneventful endocapsular lens extractions were performed through a 2.0 mm capsulorrhexis using a 20 gauge phaco needle with Healon 5^{®} brand hyaluronic acid for anterior chamber maintenance. During phaco, the capsulorrhexis stretched and a 4.5. mm silicone plug was positioned under the capsulorrhexis. In 71719 OD, the polysiloxane polymer material containing a few small bubbles was injected with a 20 gauge cannula on a 3 cc syringe. In 71719 OS, a SI-40NB IOL devoid of haptics was inserted into the capsule bag with the Silver Insertion System and the polysiloxane polymer material was injected into capsule bag in front of the IOL.

Rabbits 71565 and 71566: In these 3 year old large rabbits, the lens nucleus density was estimated to be 3-4+. Endocapsular lens extractions were performed through a 2.0 mm capsulorrhexis using a 21 gauge Shark Tooth Phaco needle with Healon 5^{®} brand hyaluronic acid for anterior chamber maintenance. The Phaco time for 71565 OD was 20 minutes. The Phaco time for 71565 OS was 18 minutes. Following lens removal, rabbit 71565 was euthanized. The Phaco time for 71566 OD was 13 minutes. After multiple attempts a 4.5 mm silicone plug was placed under the stretched capsulorrhexis and 0.3 cc of the polysiloxane polymer material was injected into the capsule bag. Slit lamp biomicroscopy was performed on Days 0, 1, 7, 15, 18, 26, 44, 74 and 107.

### Results

Immediately postoperative, all wounds were intact. Trace conjunctival injection, corneal haze and edema were noted at day 1 which resolved by one week. Trace anterior chamber flare was seen at day 1-5 which resolved by 2 weeks. Mild posterior synechiae to the capsulorrhexis was seen in all eyes. Severe synechiae was noted in one eye at days 7-26. The anterior capsulorrhexis was sealed by the 4.5 mm silicone plug and/or the Acuvue contact lens throughout the study, except in 2 eyes. In rabbit eye 71719 OS (SI-40 IOL) the polysiloxane polymer material and S140NB IOL was noted to extrude into the anterior chamber at Day 26.

### Results for Rabbit # 71719 OD:

| **Days Post OP j** | **Con** | **Cornea** | **AC Cells** | **AC Flare** | **AC Fibri n** | **Post Syn** | **Patch Position** | **Anterior Capsule Clarity** | **Poly-Posterior siloxane Capsule Polymer** |
|---|---|---|---|---|---|---|---|---|---|
| Day 1 | 1 | 0 | Sil drop | 1 | 0 | 0 | OK | Clear | clear 1+ haze |
| Day 7 | 0 | 0 | Sil drop | 0 | 0 | 0 | OK | Striae 2+ | clear 1+ haze |
| Day 15 | 0 | 0 | Sil drop | 0 | 0 | 0 | OK | Striae 2+ | clear 1+ haze |
| Day 18 | 0 | 0 | Sil drop | 0 | 0 | 15 | OK | Striae 2+ | clear Limited view |
| Day 26 | 0 | 0 | Sil drop | 0 | 0 | 5 | OK | Striae 2+ | clear Clear growth ant cap, post cap less clear |
| Day 44 | 0 | 0 | Sil drop | 0 | 0 | 5 | Recessed, clear glob ant to | Large cpx scar | clear Clear growth ant cap, post cap less clear |
| Day 74 | 0 | 0 | Sil drop | 0 | 0 | 0 | OK | Folds | clear Clear growth ant cap, post cap less clear |
| Day 107 | 0 | 0 | Sil drop | 0 | 0 | 0 | OK | Linear scar | clear Clear growth ant cap, post cap 3-4+ opaque |

### Results for Rabbit # 71719 OS:

| **Days Post OP** | **Conj** | **Cornea** | **AC Cells** | **AC Flare** | **AC Fibrin** | **Post Syn** | **Patch Position** | **Anterior Capsule** | **Polysiloxane Polymer Clarity** | **Posterior Capsule** |
|---|---|---|---|---|---|---|---|---|---|---|
| Day 1 | 1 | 0 | 0 | 1 | 0 | 0 | OK | 0 | clear | Clear IOL |
| Day 7 | 0 | 0 | 0 | 1 | 0 | 300 | OK | 1+ haze, folds | clear | 1+ haze, IOL on pc |
| Day 15 | 0 | 0 | 0 | 0 | 0 | 45 | OK | 1+ haze, folds | clear | 1+ haze, IOL on pc |
| Day 18 | 0 | 0 | 0 | 0 | 0 | 20 | OK | scar | clear | 1 + haze, IOL on pc |
| Day 26 | 0 | 0 | NV | NA | NA | 360 | NA | NA | NO. | polysiloxane polymer material & IOL in ant chamber |
| Day 44 | 0 | 0 | NV | NA | NA | NA | NA | NA | NA | polysiloxane polymer material & IOL in ant chamber |
| Day 74 | 0 | 0 | NV | NA | NA | NA | NA | NA | NA | polysiloxane polymer material & IOL in ant chamber |
| Day 107 | 0 | 0 | | NA | NA | NA | NA | NA | NA | polysiloxane polymer material & IOL in ant chamber |

### Results for Rabbit # 71566 OD:

| **Days Post OP** | **Conj** | **Cornea** | **AC Cells** | **AC Flare** | **AC Fibrin** | **Post Syn** | **Patch Position** | **Anterior Capsule** | **Polysiloxane Polymer Clarity** | **Posterior Capsule** |
|---|---|---|---|---|---|---|---|---|---|---|
| Day 1 | 1 | 1 | 0 | 1 | 0 | 0 | OK | 0 | clear | Clear with few bubbles |
| Day 7 | 0 | 0 | 0 | 0 | 0 | 5 | OK | 0 | clear | 1+ irregular surface |
| Day 15 | 0 | 0 | 0 | 0 | 0 | 5 | OK | Patchy haze | clear | Folds |
| Day 18 | 0 | 0 | 0 | 0 | 0 | 0 | OK | 1+ haze | clear | 1+ scar |
| Day 26 | 0 | 0 | 0 | 0 | 0 | 0 | OK | Thin patchy growth | clear | Patchy growth, cobble appearance |
| Day 44 | 0 | 0 | 0 | 0 | 0 | 0 | OK | 1+ haze | clear | Lens regrowth surrounds polysiloxane polymer material |
| Day 74 | 0 | 0 | 0 | 0 | 0 | 0 | OK | 1+ haze | clear | 2+ growth, cobble appearance, 2+ haze |
| Day 107 | 0 | 0 | 0 | 0 | 0 | 0 | OK | Sil droplets | clear | 2+ growth, 2+ haze |

### Discussion of Example 1a Study Results

### Combined Soft Foldable Lens and Polysiloxane Polymer Material

In two separate eyes a SI40NB IOL or an Acuvue contact lens were placed intracapsularly prior to injection of the polysiloxane polymer material. The S140NB IOL was noted to rest against the posterior capsule and was associated with trace anterior and posterior capsule haze immediately postoperative. As time progressed the polysiloxane polymer material and the S140NB IOL were extruded into the anterior chamber for no apparent reason. The Acuvue^{®} contact lens was noted to rest against the clear anterior capsule for the two month follow-up period. It is of note that the Acuvue^{©}/polysiloxane polymer material eye had the only clear anterior capsule (devoid of haze, striae or folds) in the 3 studies. It is possible that direct contact of silicone materials to the capsule and/or lens epithelial cells contributes to capsular haze; striae and/or folds.

### Hard Lens Removal Through Small Capsulorrhexis

Three year old large rabbits have a large lens with a nuclear density estimated to be 3-4+. These lenses are thought to be very similar to the adult cataractous lens with a 3-4+ nuclear density. In this study, the hard lens was able to be removed through a 2.0 mm capsulorrhexis using a 21 gauge Shark Tooth Phaco needle. However, the phaco time was exceedingly long, averaging from 13 to 20 minutes. It is important to note that these lenses were much larger than the human lens (maybe as much as twice the size of the human lens) and therefore one would anticipate being able to reduce the total phaco time to 5 to 10 minutes in the human eye.

### Example 1b

This study evaluated the implantation of an Acuvue^{®} contact lens in combination with the polysiloxanes polymer material. The New Zealand white female rabbit (Rabbit 71891) was about 3-4 months old at time of surgery and weighed 2.6 kg.

Rabbit 71891 OD, OS: Uneventful endocapsular lens extractions were performed through a 2.0 (OD) to 2.5mm (OS) capsulorrhexis using a 20 gauge phaco needle with Healon 5^{®} brand hyaluronic acid for anterior chamber maintenance. During phaco the capsulorrhexis stretched and a 4.5 mm silicone plug was positioned under the capsulorrhexis. In the right eye, an Acuvue^{®} contact lens was cut to 6.5 mm and placed into the capsule bag with forceps. The polysiloxane polymer material (0.25 cc) was then injected under the contact lens which assisted the silicone plug in preventing polysiloxane polymer leakage. In the left eye, the polysiloxane polymer material (0.15 cc) was injected into the capsule bag with slight leakage.

Slit lamp biomicroscopy was performed Days 0, 21, 38 and 53. Immediately postoperative, all wounds were intact. Trace to mild corneal haze and edema were noted day 1 and resolved by one week.

### Results for Rabbit #71891 OD

| **Days Post OP** | **Conj** | **Cornea** | **AC Cells** | **AC Flare** | **AC Fibrin** | **Post Syn** | **Patch Position** | **Anterio r Capsul e** | **Polysiloxane Polymer Clarity** | **Posterior Capsule** |
|---|---|---|---|---|---|---|---|---|---|---|
| Day 21 | 0 | 2 | 0 | 0 | 0 | 20 | CL in place | 1 | clear | Striae, 1+ haze |
| Day 38 | 0 | 0 | 0 | 0 | 0 | 10 | CL in place | 0 | clear | 1+ growth , cobble appearance |
| Day 53 | 0 | 0 | 0 | 0 | 0 | 90 | CL in place | 0 | clear | 3+ pco on 100% pc, 3+ opaque |

### Results for Rabbit #71891 OS

| **Days Post OP** | **Conj** | **Cornea** | **AC Cells** | **AC Flare** | **AC Fibrin** | **Post Syn** | **Patch Position** | **Anterior Capsule** | **Polysiloxane Polymer Clarity** | **Posterior Capsule** |
|---|---|---|---|---|---|---|---|---|---|---|
| Day 21 | 0 | 1 | 0 | 0 | 0 | 15 | OK | 1+ haze | clear | 2+ straie, 1+ haze |
| Day 38 | 0 | 0 | 0 | 0 | 0 | 15 | OK | 1+ haze | clear | 2+ growth, 2+ haze |
| Day 53 | 0 | 0 | 0 | 0 | 0 | 90 | OK | 0 | clear | 3+ pco on 100% pc, 3+ opaque |

### Example 1c

Example 1 c was designed to quantitate the amount of polysiloxane polymer material required to fill the capsule bag of 3 month old New Zealand white rabbits. The rabbits used in this study were 3-4 months old and weighed 2.5-2.6 kg.

Rabbits 72823 OD, 72824 OD, 72825 OD: Uneventful endocapsular lens extractions were performed through a 3.0 capsulorrhexis using a 21 gauge phaco needle with Healon 5^{®} brand hyaluronic acid for anterior chamber maintenance. A 4.5 mm silicone plug was placed into the capsular bag and 0.2 cc, 0.1 cc and 0.2 cc of the polysiloxane polymer material was injected into the capsule bag of rabbit 72823 OD, 72824 OD and 72825 OD, respectfully. Minimal air bubbles and polysiloxane polymer leakage where noted. Slit lamp biomicroscopy was performed on Days 0, 5, 26 and 57.

Immediately postoperative, all wounds were intact. Trace anterior chamber flare was seen at day 1-5 which resolved by 2 weeks. Mild anterior chamber fibrin was noted in one eye at day 1 and a small polysiloxane polymer material bubble was seen in one eye. In rabbit 72824 OD the silicone plug and polysiloxane polymer material also protruded into the anterior chamber at day 26.

### Results for Rabbit #72823 OD

| **Days Post OP** | **Conj** | **Cornea** | **AC Cells** | **AC Flare** | **AC Fibrin** | **Post Syn** | **Patch Position** | **Anterior Capsule** | **Polysiloxane Polymer Clarity** | **Posterior Capsule** |
|---|---|---|---|---|---|---|---|---|---|---|
| Day 5 | 0 | 0 | 0 | 1 | 0 | 0 | OK | 0 | underfilled | Fibrous band at polysiloxane polymer |
| Day 26 | 0 | 0 | 0 | 0 | 0 | 15 | OK | 0 | fair | 2+growth, cobble 70% of pc |
| Day 57 | 0 | 0 | 0 | 0 | 0 | polysiloxane polymer mat prolapse | moved | 1+cloudy, 1+ growth | clear | 2+ growth, 2-3+ haze |

### Results for Rabbit #72824 OD

| **Days Post OP** | **Conj** | **Cornea** | **AC Cells** | **AC Flare** | **AC Fibrin** | **Post Syn** | **Patch Position** | **Anterior Capsule** | **Polysiloxane Polymer Clarity** | **Posterior Capsule** |
|---|---|---|---|---|---|---|---|---|---|---|
| Day 5 | 0 | 0 | polysiloxane polymer on iris | 1 | 2 | 15 | OK | ?? | clear | |
| Day 26 | 0 | 0 | 0 | 0 | 0 | 15 | Protruding into ac | scars | Protruding into ac | 1+ haze |
| Day 57 | 0 | 0 | NA | NA | NA | NA | NA | NA | Protruding into ac | |

### Results for Rabbit #72825 OD

| **Days Post OP** | **Conj** | **Cornea** | **AC Cells** | **AC Flare** | **AC Fibrin** | **Post Syn** | **Patch Position** | **Anterior Capsule** | **Polysiloxane Polymer Clarity** | **Posterior Capsule** |
|---|---|---|---|---|---|---|---|---|---|---|
| Day 5 | 0 | 0 | 0 | 1 | 0 | 0 | OK | 0 | clear | Fibrous band at polysiloxane polymer |
| Day 26 | 0 | 0 | 0 | 0 | 0 | 0 | OK | folds | clear | 1+growth, cobble |
| Day 57 | 0 | 0 | 0 | 0 | 0 | 5 | OK | 1+ haze | clear | 2+ growth, 2-3+ haze |

### Summary of Example 1 Results

Immediately postoperative, the capsule bags were clear and distended with the clear polysiloxane polymer material. The polysiloxane polymer material used remained clear in all studies throughout the follow-up period (for as long as 107 days). Underfilling of the capsule bag was noted in some eyes. In particular, 0.1 cc of the polysiloxane polymer material was not enough to fill the capsule bag and was associated with slippage of the silicone plug and extrusion of polysiloxane polymer material into the anterior chamber. Rather, about 0.2 cc of polysiloxane polymer material was required to fill the capsule bag of these 3-4 months old New Zealand white rabbits, weighing 2.5-2.6 kg.

Immediately postoperative, trace posterior capsule haze was noted in most eyes and gradually progressed with striae and folds also noted. Posterior capsule lens regrowth was first noted at day 26 and gradually progressed surrounding the polysiloxane polymer material. In general lens regrowth was clear anterior and peripheral to the polysiloxane polymer material and more irregular and opaque posterior to the polysiloxane polymer material. By one week varying degrees of anterior capsule haze, striae and folds were noted in all eyes except the one eye with the Acuvue^{®} lens against the anterior capsule.

In summary, the results of the described studies indicated that: (i) endocapsular lens extraction could be performed through a small 2.0 mm capsulorrhexis in the hard lens (3-4+) of 3 year old large rabbits; (ii) a clear anterior capsule devoid of haze, striae or folds was noted in the one eye with the Acuvue^{®} contact lens positioned between the anterior capsule and polysiloxane polymer material; (iii) the polysiloxane polymer material was an intralenticular lens with clear regenerative lens material surrounding it, thus creating a bilenticular lens; and (iv) the 3-4 month old rabbit requires about 0.2 cc of the polysiloxane polymer material to fill the capsule bag.

### Example 2

The following *in vivo* study was designed to evaluate lens regeneration with a polysiloxane polymer material as a flexible and biocompatible polymeric scaffold for lens regeneration following endocapsular lens extraction with and without the injection of additional biodegradable materials.

### General Materials

The polysiloxane polymer material used in the presently described study was obtained from AMO Groningen B.V. (Groningen, Holland) and corresponded to composition AS4-11, CS0402014. Silicone plugs (2.7 mm and 4.5mm) were also obtained from AMO Groningen B.V. (Groningen, Holland). Healon^{®} brand hyaluronic acid and Healon 5^{®} brand hyaluronic acid were obtained from AMO USA, Inc. (Santa Ana, California, USA).

### General Methods

Six three-month old female New Zealand white rabbits weighing about 2.5-2.6 kg at the time of surgery were used. The general health and acceptability of the animals was established prior to surgery. Animals were treated in accordance with USDA guidelines and the ARVO Resolution on the Use of Animals in Research.

Rabbits were anesthetized with about 5 mg/kg xylazine and about 50 *mg/kg ketamine HCl, intramuscularly. The surgical eye was dilated with 1% cyclopentolate and 10% phenylephrine; eyelashes were trimmed; and the ocular area was disinfected with povidone iodine. A wire lid speculum was inserted to retract the lids, and a corneal incision was made at 12:00 with a 2.85 mm keratome. Healon 5^{®} brand hyaluronic acid was injected to maintain anterior chamber depth and an about 2-3 mm continuous curvilinear capsulorrhexis was performed. A 20 gauge phacoemulsification tip was inserted through the corneal wound and endocapsular lens extraction was performed by phacoemulsification and irrigation/aspiration with balanced salt solution (BSS). Considerable care was taken to remove all lens cortical material by diligent irrigation and aspiration. A 2.7 mm or 4.5 mm silicone plug was inserted into the capsule bag and maneuvered behind the anterior capsulotomy. In one eye following lens extraction, Healon^{®} brand hyaluronic acid was injected into the capsule bag to coat the anterior and posterior capsule followed by injection of the polysiloxane polymer material. In the opposite eye, the Healon^{®} brand hyaluronic acid was not administered and the polysiloxane polymer material alone was injected into the empty capsule bag using a 20 gauge cannula. About 0.07cc to about 0.14 cc of the polysiloxane polymer material was injected into the capsule bag of the rabbits. Minimal air bubbles and polysiloxane polymer leakage were noted during administration.

In rabbit eye #74718 OD, epinephrine was added to the balanced salt solution to increase dilation. In rabbit eye #74717 OS, the capsulorrhexis stretched during phacoemulsification and a double patch consisting of a 4.5 mm silicone plug and a 24 hour collagen patch was placed in this eye. A 4.5 mm silicone plug was placed in rabbit eye #74719 to seal the capsulotomy. In one Healon^{®} HAlpolysiloxane polymer rabbit eye, #74725 OD, the polysiloxane polymer material leaked into the anterior chamber. The Healon^{®} brand hyaluronic acid was injected after polysiloxane polymer material leaked out and before new polysiloxane polymer material was reinjected.

At the completion of these surgical procedures, corneal incisions were closed with 10-0 nylon sutures. 0.25 ml (20 mg) of gentamicin and 0.1 ml of dexamethasone (2mg) was injected subconjunctivally at the end of each surgery and every 3 days thereafter for two weeks.

Slit lamp biomicroscopy was performed on: Day 0, 2, 15, 20, 29, 42 and 50. The overall positional stability of the silicone plug was evaluated based on the slit lamp observation of placement in the capsule bag. Observations of conjunctiva, corneal pathology, anterior chamber cells, flare and fibrin, posterior synechiae, capsular bag shape, and percent lens regrowth in the capsule bag were recorded. All slit lamp findings were graded on a scale of 0 to 4 (0=none, 1+ = trace, 2+ = mild, 3+ = moderate, 4+ = severe) as described in further detail in Example 1. Further, throughout the experimental period, rabbits were observed for any abnormal clinical signs, including any abnormal ocular findings such as pain, excessive hyperemia or discharge. All observations were recorded on the Slit Lamp Examination form. Grading for corneal edema/cloudiness; cells in the anterior chamber; anterior chamber flare; fibrin in the anterior chamber; and posterior synechia was performed as described in relation to Example 1.

At the end of the study, animals were euthanized by an injection of sodium pentobarbital into the marginal ear vein. Rabbit eyes 74717 OU and 74718 OU had the polysiloxane polymer material removed surgically before all eyes were placed in formalin.

### Results

Immediately postoperative, all wounds were intact. Conjunctival injection, corneal edema and haze, anterior chamber cells and flare and anterior chamber fibrin were mild and similar in both groups. Mean conjunctival scores were 0.8±0.4 and 1.0±0.8, mean corneal edema and haze scores were 0.8±0.4 and 0.7±0.4, mean anterior chamber cell scores were 0.3±0.5 and 0.2±0.4, mean flare scores were 1.2±0.4 and 1.0±0.6 and mean anterior chamber fibrin scores were 1.2±0.4 and 0.5±0.5 in the polysiloxane polymer and Healon^{®} HA/polysiloxane polymer groups, respectfully. All inflammatory signs resolved by day 15. One eye in the Healon^{®}HA/polysiloxane polymer group had a drop of silicone material in the anterior chamber. Relevant results are summarized in the following tables:

| **Anterior Chamber Cells-Polysiloxane Polymer:** | | | | | | | | | **Anterior Chamber Cells-Healon^{®}/polysiloxane Polymer:** | | | | | | | |
|---|---|---|---|---|---|---|---|---|---|---|---|---|---|---|---|---|
| ID/Eye | D0 | D2 | D15 | D20 | D 29 | D 42 | D 50 | | ID/Eye | D 0 | D2 | D15 | D 20 | D 29 | D 42 | D50 |
| **74717 OD** | 0 | 1 | 0 | 0 | 0 | 0 | 0 | | **74717 OS** | 0 | 0 | 0 | 0 | 0 | 0 | 0 |
| **74718 OD** | 0 | 0 | 0 | 0 | 0 | 0 | 0 | | 7**4718 OS** | 0 | 1 | 0 | 0 | 0 | 0 | 0 |
| **74719 OD** | 0 | 0 | 0 | 0 | 0 | 0 | 0 | | **74719 OS** | 0 | 0 | 0 | 0 | 0 | 0 | 0 |
| **74725 OS** | 0 | 1 | 0 | 0 | 0 | 0 | 0 | | **74725 OD** | 0 | 0 | 0 | 0 | 0 | 0 | 0 |
| **74726 OD** | 0 | 0 | 0 | 0 | 0 | 0 | 0 | | **74726 OS** | 0 | 0 | 0 | 0 | 0 | 0 | 0 |
| **74727 OD** | 0 | 0 | 0 | 0 | 0 | 0 | 0 | | **74727 OS** | 0 | 0 | 0 | 0 | 0 | 0 | 0 |
| **Mean** | **0** | **0.3** | **0** | **0** | **0** | **0** | **0** | | **Mean** | **0** | **0.2** | **0** | **0** | **0** | **0** | **0** |
| **SE** | **0.0** | **0.2** | **0.0** | **0.0** | **0.0** | **0.0** | **0.0** | | **SE** | **0.0** | **0.2** | **0.0** | **0.0** | **0.0** | **0.0** | **0.0** |
| **SD** | **0.0** | **0.5** | **0.0** | **0.0** | **0.0** | **0.0** | **0.0** | | **SD** | **0.0** | **0.4** | **0.0** | **0.0** | **0.0** | **0.0** | **0.0** |

| **Anterior Chamber Flare-Polysiloxane Polymer:** | | | | | | | | | **Anterior Chamber Flare-Healon^{®}/polysiloxane Polymer:** | | | | | | | |
|---|---|---|---|---|---|---|---|---|---|---|---|---|---|---|---|---|
| ID/Eye | D 0 | D2 | D15 | D 20 | D 29 | D 42 | D 50 | | ID/Eye | D 0 | D2 | D 15 | D 20 | D29 | D 42 | D 60 |
| **74717 OD** | 0 | 1 | 0 | 0 | 0 | 0 | 0 | | **74717 OS** | 0 | 2 | 0 | 0 | 0 | 0 | 0 |
| **OD 74718** | 0 | 1 | 0 | 0 | 0 | 0 | 0 | | **OS** | **74718** 0 | 1 | 0 | 0 | 0 | 0 | 0 |
| **74719 OD** | 0 | 2 | 0 | 0 | 0 | 0 | 0 | | **74719 OS** | 0 | 1 | 0 | 0 | 0 | 0 | 0 |
| **74725 OS** | 0 | 1 | 0 | 0 | 0 | 0 | 0 | | **74725 OD** | 0 | 0 | 0 | 0 | 0 | 0 | 0 |
| **74726 OD** | 0 | 1 | 0 | 0 | 0 | 0 | 0 | | **74726 OS** | 0 | 1 | 0 | 0 | 0 | 0 | 0 |
| **74727 OD** | 0 | 1 | 0 | 0 | 0 | 0 | 0 | | **74727 OS** | 0 | 1 | 0 | 0 | 0 | 0 | 0 |
| **Mean** | **0** | **1.2** | **0** | **0** | **0** | **0** | **0** | | **Mean** | **0** | **1.0** | **0** | **0** | **0** | **0** | **0** |
| **SE** | **0.0** | **0.2** | **0.0** | **0.0** | **0.0** | **0.0** | **0.0** | | **SE** | **0.0** | **0.2** | **0.0** | **0.0** | **0.0** | **0.0** | **0.0** |
| **SD** | **0.0** | **0.4** | **0.0** | **0.0** | **0.0** | **0.0** | **0.0** | | **SD** | **0.0** | **0.6** | **0.0** | **0.0** | **0.0** | **0.0** | **0.0** |

| **Anterior Chamber Fibrin-Polysiloxane Polymer:** | | | | | | | | | **Anterior Chamber Fibrin-Healon^{®}/polysiloxane Polymer:** | | | | | | | |
|---|---|---|---|---|---|---|---|---|---|---|---|---|---|---|---|---|
| ID/Eye | D 0 | D2 | D 15 | D 20 | D 29 | D42 | D 50 | | ID/Eye | D 0 | D2 | D15 | D 20 | D 29 | D42 | D 50 |
| **74717 OD** | 0 | 1 | 0 | 0 | 0 | 0 | 0 | | **74717 OS** | 0 | 0 | 0 | 0 | 0 | 0 | 0 |
| **74718** 0 **OD** | | 2 | 0 | 0 | 0 | 0 | 0 | | **74718** 0 **OS** | | 1 | 0 | 0 | 0 | 0 | 0 |
| **74719 OD** | 0 | 1 | 0 | 0 | 0 | 0 | 0 | | **74719 OS** | 0 | 0 | 0 | 0 | 0 | 0 . | 0 |
| **74725 OS** | 0 | 1 | 0 | 0 | 0 | 0 | 0 | | **74725 OD** | 0 | 0 | 0 | 0 | 0 | 0 | 0 |
| **74726 OD** | 0 | 1 | 0 | 0 | 0 | 0 | 0 | | **74726 OS** | 0 | 1 | 0 | 0 | 0 | 0 | 0 |
| **74727 OD** | 0 | 1 | 0 | 0 | 0 | 0 | 0 | | **74727 OS** | 0 | 1 | 0 | 0 | 0 | 0 | 0 |
| **Mean** | **0** | **1.2** | **0** | **0** | **0** | **0** | **0** | | **Mean** | **0** | **0.5** | **0** | **0** | **0** | **0** | **0** |
| **SE** | **0.0** | **0.2** | **0.0** | **0.0** | **0.0** | **0.0** | **0.0** | | **SE** | **0.0** | **0.2** | **0.0** | **0.0** | **0.0** | **0.0** | **0.0** |
| **SD** | **0.0** | **0.4** | **0.0** | **0.0** | **0.0** | **0.0** | **0.0** | | **SD** | **0.0** | **0.5** | **0.0** | **0.0** | **0.0** | **0.0** | **0.0** |

As can be seen in the following tables, trace posterior synechiae was first noted at day 2 in 5 of the 6 eyes in both groups and gradually diminished by the end of the study at day 50:

| **Posterior Polysiloxane Synechiae Polymer: (degrees)-** | | | | | | | | | **Posterior Healon^{®}/polysiloxane Synechiae (degrees)- Polymer:** | | | | | | | |
|---|---|---|---|---|---|---|---|---|---|---|---|---|---|---|---|---|
| ID/Eye | D 0 | D2 | D 16 | D 20 | D29 | D 42 | D 50 | | ID/Eye | D 0 | D 2 | D 15 | D 20 | D 29 | D 42 | D 50 |
| **74717 OD** | 0 | 10 | 5 | 5 | 5 | 5 | 5 | | **74717 OS** | 0 | 10 | 5 | 5 | 5 | 0 | 0 |
| **74718 OD** | 0 | 15 | 10 | 5 | 5 | 0 | 0 | | **74718 OS** | 0 | 15 | 5 | 5 | 0 | 0 | 0 |
| **74719 OD** | 0 | 0 | 5 | 0 | 0 | 0 | 0 | | **74719 OS** | 0 | 5 | 0 | 0 | 0 | 0 | 0 |
| **74725 OS** | 0 | 5 | 5 | 10 | 10 | 10 | 0 | | **74725 OD** | 0 | 0 | 0 | 0 | 0 | 0 | 0 |
| **74726 OD** | 0 | 10 | 5 | 5 | 5 | 0 | 0 | | **74726 OS** | 0 | 15 | 5 | 5 | 0 | 0 | 0 |
| **74727 OD** | 0 | 15 | 5 | 5 | 0 | 0 | 0 | | **74727 OS** | 0 | 10 | 0 | 0 | 0 | 0 | 0 |

Mean capsulorrhexis size was 1.9±0.2 in the polysiloxane polymer group and 2.3±0.5 in the Healon^{®} HAlpolysiloxane polymer group. Immediately postoperative, the anterior capsulotomy was sealed by the silicone plug in all eyes and the collagen patch in the one eye of the Healon^{®} HAlpolysiloxane polymer group. The anterior capsulotomies remained closed in all eyes throughout the study.

Immediately postoperative, the capsule bags were distended with the polysiloxane polymer material. The clear polysiloxane polymer material filled about 60% to about 100% of the capsule bag in the polysiloxane polymer and Healon^{®} HAlpolysiloxane polymer groups. In both the polysiloxane polymer and Healon^{®} HAlpolysiloxane polymer groups, 0.1-0.14 cc of polysiloxane polymer was required to fill the capsule bag while 0.07-0.08 cc polysiloxane polymer filled only 60-70% of the capsule bag:

| **Polysiloxane Polymer Clarity/ %filling of Capsule Bag-Polysiloxane Polymer:** | | | | | | | | | **Polysiloxane Polymer Clarity/ %filling of Capsule Bag-Healon^{®}/polysiloxane Polymer:** | | | | | | | |
|---|---|---|---|---|---|---|---|---|---|---|---|---|---|---|---|---|
| ID/Eye | ACL dose (cc) | D2 | D 15 | D 20 | D 29 | D 42 | D 50 | | ID/Eye | ACL dose (cc) | D2 | D 15 | D 20 | D 29 | D 42 | D 50 |
| **74717 OD** | 0.08 | clear | Clear/ 70% | Clear/ 70% | Clear/ 70% | Clear/ 70% | Clear/ 70% | | **74717 OS** | **0.1** | clear | Clear/ 80% | Clear/ 80% | Clear/ 80% | Clear/ 80% | Clea_{d} 80% |
| **74718 OD** | 0.14 | clear | Clear/ 100% | Clear/ 100% | Clear/ 100% | Clear/ 100% | Clear/ 100% | | **74718 OS** | 0.1 | clear | Clear/ 100% | Clear/ 100% | Clear/ 100% | Clear/ 100% | Clear/ 100% |
| **74719 OD** | 0.08 | clear | Clear/ 70% | Clear/ 70% | Clear/ 70% | Clear/ 70% | Clear/ 70% | | **74719 OS** | 0.1 | clear | Clear/ 100% | Clear/ 100% | Clear/ 100% | Clear/ 100% | Clear/ 100% |
| **74725 OS** | 0.1 | clear | Clear/ 100% | Clear/ 100% | Clear/ 100% | Clear/ 100% | Clear/ 100% | | **74725 OD** | 0.1 | clear | clear | clear | clear | clear | Clear/ globs |
| **74726 OD** | 0.1 | clear | Clear/ 70% | Clear/ 70% | Clear/ 70% | Clear/ 70% | Clear/ 70% | | **74726 OS** | 0.07 | clear | Clear/ 60% | Clear/ 60% | Clear/ 60% | Clear/ 60% | Clear/ 60% |
| **74727 OD** | 0.1 | clear | Clear/ 60% | Clear/ 60% | Clear/ 60% | Clear/ 60% | Clear/ 60% | | **74727 OS** | 0.1 | clear | Clear/ 90% | Clear/ 90% | Clear/ 90% | Clear/ 90% | Clear/ 90% |

Immediately postoperative at day 5, the anterior capsule was clear in all eyes in both groups. By day 15, mild to moderate fibrosis was seen in all eyes in the polysiloxane polymer only group. Fibrosis remained stable in 4 of 6 eyes and gradually progressed to moderate in 2 eyes. In the Healon^{®} HAlpolysiloxane polymer group, the anterior capsule remained clear without folds, striae or fibrosis in 4 of 6 eyes throughout the 50 day follow-up period. In one eye where surgery was complicated by polysiloxane polymer leakage and Healon^{®} brand hyaluronic acid was injected before and after polysiloxane polymer injection there was moderate fibrosis of the anterior capsule. In another eye, one fibrotic strand extended lineally from the capsulotomy:

| **Anterior Capsule Folds/striae/fibrosis -Folds/striae/fibrosis - Polysiloxane Polymer:** | | | | | | | | **Anterior Capsule Healon**^{®}**/polysiloxane Polymer:** | | | | | | |
|---|---|---|---|---|---|---|---|---|---|---|---|---|---|---|
| ID/Eye | D2 | D15 | D20 | D 29 | D42 | D50 | | ID/Eye | D2 | D 15 | D 20 | D 29 | D 42 | D 50 |
| **74717 OD** | N | 1+ | 1+ | 2+ | 2+ | 2+ | | **74717 OS** | N | N | N | N | N | N |
| **74718 OD** | N | 1+ | 1+ | 1+ | 1+ | 1**+** | | **74718 OS** | N | N | N | N | N | N |
| **74719 OD** | N | 2+* | 2+ | 2+ | 2+ | 2+ | | **74719 OS** | N | N | N | N | N | N |
| **74725 OS** | N | 1+ | 1+ | 1+ | 1+ | 1+ | | **74725 OD** | N | 2+ | 2+ | 2+ | 2+ | 2+ |
| **74726 OD** | N | 1+ | 2+ | 2+ | 2+ | 2+ | | **74726 OS** | N | N | N | N | N | N |
| **74727 OD** | N | 1+* | 1+ | 1 + | 1 + | 1 + | | **74727 OS** | N | 1+ | 1+ | 1+* | 1+* | 1+* |

| | | | | | | | | | | | | | | |
|---|---|---|---|---|---|---|---|---|---|---|---|---|---|---|
| * adhesion to posterior capsule superiorly | | | | | | | | | | | | | | |

Lens regrowth was first noted in one eye each at day 20 in the polysiloxane polymer group and at day 15 in the Healon^{®} HAlpolysiloxane polymer group. By day 42, 5 to 10% lens regrowth was noted in all eyes in the polysiloxane polymer group and 5 to 20% lens regrowth was noted in the Healon^{®} HAlpolysiloxane polymer group. In both groups lens regrowth was generally clear in the peripheral capsule bag and more opacified posterior to the polysiloxane polymer material. Lens regrowth was clear in 2 eyes and slight to mildly opaque in 4 eyes in each group:

| **Lens Regrowth (%) Polysiloxane Polymer:** | | | | | | | | **Lens Regrowth (%) Heaion**^{®}**/polysiloxane Polymer:** | | | | | | |
|---|---|---|---|---|---|---|---|---|---|---|---|---|---|---|
| ID/Eye | D 2 | D 15 | D 20 | D 29 | D 42 | D 50 | | ID/Eye | D 2 | D 15 | D 20 | D 29 | D 42 | D 50 |
| **74717 OD** | 0 | 0 | 0 | 0 | 10 2+ opacity | 5 | | **74717 OS** | 0 | 0 | 10 | 10 | 20 | 20 |
| **74718 OD** | 0 | 0 | 0 | 0 | 5-10% 2+ opacity | 5 | | **74718 OS** | 0 | 0 | 10 | 10 | 10 1+ opacity | 5 |
| **74719 OD** | 0 | ac adhesion | 1 | 5 | 5 limited view | 5 | | **74719 OS** | 0 | 1 1+ haze | 5 | 5 | 5 2+ opacity | 5 |
| **74725 OS** | 0 | 0 | 0 | 0 | 10 | 5 | | **OD 74725** | 0 | 0 | 0 | 5 | 5 | 5 |
| **74726 OD** | 0 | 0 | 0 | 0 | 5 1+ opacity | 5 1+ opacity | | **74726 OS** | 0 | 0 | 1 | 5 | 20 1+ opacity | 5 |
| **74727 OD** | 0 | ac adhesion | 0 | 5 | 5 opacity 1+ | 5 opacity 1+ | | **74727 OS** | 0 | 0 | 1 | 1 | 5 opacity 2+ | 5 opacity 1+ |

### Discussion and Summary of Example 2 Results

The study described in Example 2 was designed to evaluate lens regeneration with polysiloxane polymer material as a flexible and biocompatible polymeric lens following endocapsular lens extraction in young New Zealand white rabbits with or without the inclusion of an additional biocompatible material. Overall the inflammatory response to lens extraction and polysiloxane polymer injection was mild and resolved by the second postoperative visit at 2 weeks in both groups. Mild posterior synechiae to the capsulotomy site gradually diminished and the anterior capsulotomy remained closed throughout the study.

About 0.1 to 0.14 cc of the clear polysiloxane polymer material was required to completely fill the capsule bag. Immediately postoperative, the capsule bag and polysiloxane polymer material were clear in all eyes in both groups. By the second examination at 2 weeks, anterior capsule fibrosis was noted in all eyes in the polysiloxane polymer only group. The fibrosis remained stable in 4 eyes and gradually increased in 2 eyes. In contrast the anterior capsule remained clear in 4 eyes in the Healon^{®} HA/polysiloxane polymer group. This suggests that the Healon^{®} brand hyaluronic acid may have an antifibrotic effect and may also prevent contact between the polymer and the capsule.

Peripheral and posterior capsule lens regrowth surrounding the polysiloxane polymer material was first noted in one eye at day 20 in the polysiloxane polymer group and at day 15 in the Healon^{®} HA/polysiloxane polymer group. Lens regrowth gradually developed in all eyes by day 42 and was generally clear in the peripheral capsule bag and more opacified posterior to the polysiloxane polymer material. The full development of lens regeneration around the polysiloxane polymer material was limited by the early termination of the study for administrative reasons.

Thus, in the described study, limited lens regeneration was observed as early as 15 days surrounding the polysiloxane polymer material scaffold following endocapsular lens extraction in 3 month old New Zealand white rabbits. Overall lens regrowth was clear anterior and in the periphery and slightly opaque posterior the polysiloxane polymer material. The administration of Healon^{®} brand hyaluronic acid prior to injection of the polysiloxane polymer material was associated with significantly less anterior capsule fibrosis.

In one embodiment of the polysiloxane polymers used in accordance with the present invention, the polysiloxane polymer has functional acryl groups that can be obtained from a polymer having the general formula: 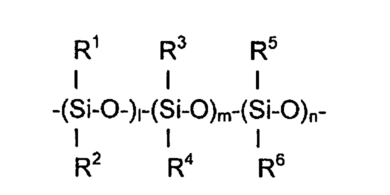 wherein R¹ and R² are independently C₁ -C₆ alkyl; R³ is phenyl; R⁴ is phenyl or C₁ - C₆ alkyl; R⁵ is CF₃ (CH₂)ₓ wherein x is 1-5; R⁶ is C₁ -C₆ alkyl or fluoroalkyl; 1 is in the molar fraction range of 0 to 0.95; m is in the molar fraction range of 0 to 0.7; and n is in the molar fraction range of 0 to 0.65, the polymer having functional acryl groups at the terminal ends thereof. In one embodiment, m is in the molar fraction range of from greater than 0 to 0.7; and n is in the molar fraction range of from greater than 0 to 0.65.

In another embodiment R¹ is methyl, that R² is methyl, R⁴ is phenyl, that x is 2, either independently, or in combination.

According to these alternatives, in one embodiment R⁶ is methyl. According to another embodiment, the polysiloxane is a polymer of diphenyl or phenylalkyl siloxane and dialkyl siloxane with terminal acryl groups. According to further embodiments, the polysiloxane is a polymer of diphenyl or phenylalkyl siloxane and trifluoroalkyl(alkyl)siloxane, or a terpolymer or higher order polymer of diphenyl and/or phenylalkyl siloxane, dialkyl siloxane and trifluoroalkyl alkyl siloxane. According to another embodiment, polysiloxane is an acryl-terminated terpolymer of dimethyl siloxane, diphenyl siloxane or phenylmethyl siloxane and 3,3,3-trifluoropropylmethyl siloxane. In certain embodiments the polysiloxanes comprise at least about 4 mol % of trifluoropropylmethyl siloxane and about 1 to about 50 mol % of diphenylsiloxane and/or phenylmethylsiloxane. In other embodiments the polysiloxanes comprise about 4 to about 65 mol % trifluoropropylmethyl siloxane, and about 1 to about 50 mol % of diphenylsiloxaue and dimethylsiloxane monomer units. One suitable acryl-terminated polysiloxane composition comprises about 28 mol % trifluoropropylmethyl siloxane, about 4 mol % diphenyl siloxane and dimethyl siloxane monomer units.

Polysiloxane polymers used in accordance with the present invention are formed into an injectable lens material having a suitable viscosity to be injected through standard cannula with an 18 Gauge needle or finer. For this purpose the material generally will have a viscosity lower than about 60 000 cSt or below about 8000 cSt for being readily injectable through a 21 Gauge needle. Injectable lens materials used in accordance with the present invention can also optionally comprise a photoinitiator and/or a crosslinking agent, which itself can be siloxane oligoimer or polymer having functional groups and further physiologically or ophthalmologically acceptable additives necessary for producing a lens.

A method of the *in vivo* production of an IOL, can comprise the steps of preparing an polysiloxane polymer having functional acryl groups; mixing the polymer and a photoinitiator, in one embodiment a medically acceptable blue light photoinitiator, into a composition; injecting the composition into the capsular bag of the eye; and initiating a polymerization reaction to create a lens in the capsular bag. Optionally, the elastomer can also comprise an UV absorbing compound or other conventional additives known to those skilled in the art. A special advantage of the materials used in accordance with the present invention is that the incorporation of a fluoroalkyl siloxane enables materials of high specific gravity to be produced

Examples of specific procedures for creating polysiloxane polymers useful in accordance with the present invention can be found in United States Patent Number 6,737,496 .

As suggested by Example 2, hyaluronic acid can also be administered in conjunction with the polymers used in accordance with the present invention. Administration of hyaluronic acid can be beneficial in wound healing. Further, fetal wounds that heal without scar formation have an extracellular matrix that is rich in hyaluronic acid. In one embodiment of the present invention, a viscoelastic substance such as hyaluronic acid may be used in conjunction with a polymer (in one embodiment a polysiloxane polymer) for capsule bag filling to enhance the regeneration of lenses following phacoemulsification and subsequent irrigation and aspiration of both the natural and cataractous lens and sealing of the anterior capsule. One skilled in the art will readily appreciate that hyaluronic acid compositions of varying viscosity, glycosaminoglycans (GAG's), and/or formulations thereof may be used in accordance with alternate embodiments of the present invention. By way of example, and not of limitation, suitable hyaluronic compositions may include, but are not limited to the following: Restylane^{®} OVD, Perlane^{®} OVD, a variant formulation of Healon^{®} OVD (AMO USA, Inc., Santa Ana, California), and/or compositions that include hyaluronic acid forms such as those described in U.S. Patent Nos.: 6,537,795; 6,090,596; 4,764,360; 6,086,597; 6,368,585; and 5,681,825; U.S. Patent Application Publication No. 2002/0018898 (Serial No. 09/855,923), and in European Patent Application 0760863 B1 . Any variant formulation or analogous composition of any of the aforementioned hyaluronic compounds and/or GAGs including, but not limited to hyaluronic acid forms with higher or lower molecular weights, hyaluronic acid forms at variant concentrations, chondroitin sulfate, a hyaluronic acid/chondroitin sulfate mixture, combinations of two or more of the above mentioned compositions, and/or combinations of any of the aforementioned compositions with other suitable agents may be used in accordance with alternate embodiments of the invention. Furthermore, inventive compositions may include a hyaluronic acid compound as well as any number of conventional carriers, additives, preservatives, antibiotics, therapeutic agents and the like that are generally formulated in pharmacological compositions of this nature, as will be readily appreciated by those of skill in the art. Such additional elements may, for example, promote the safety and/or efficacy of the inventive compound. Various quantities, molecular weights, concentrations, and/or forms of hyaluronic acid products may be used to improve the lens cell proliferation and differentiation. For example, a quantity between .01 to 3 cc of hyaluronic acid may be used to fill the lens capsule bag to improve the lens cell proliferation and differentiation.

In alternate embodiments, other media can be used individually or in combination to enhance the proliferation and differentiation of lens cells in accordance with the present invention; for instance, amniotic fluid, in vitro fertilization media, growth factors (e.g., BD MATRIGEL™ Basement Membrane Matrix and BD MATRIGEL™ Basement Membrane Matrix High Concentration; BD Biosciences, San Jose, CA), and/or other substances that can enhance or control the growth and proliferation of cells will be readily appreciated by one skilled in the art.

In another embodiment, lenticular tissue may be engineered using focal laser photophacocoagulation to remove excess viscoelastic substances and/or modify structure and clarity of the regenerated lens and/or bilenticular lens. As described in U.S. Patent No. 6,322,556 and U.S. Patent Application Publication Nos. 2002/0103478 (Serial No. 09/953,121) and 2006/0002981 (Serial No. 10/881,426) , laser photophacoablation (laser photoablation) has been used to partially remove ocular tissue (e.g., lens tissue) to correct vision deficiencies and to treat other vision-impairing ocular problems without causing substantial damage to the surrounding tissue regions. In the present invention, laser photophacoablation may be used to remove retained viscoelastic substances in the regenerated lens in combination with the inventive use of hyaluronic acid in combination with polymers including polysiloxane polymers.

Lens regeneration can also be enhanced in accordance with the present invention by sealing the anterior capsulotomy with one or more collagen patches. Insertion of a collagen patch may be effected during a procedure for treating ocular disease and/or correcting vision impairment, as for example, endocapsular lens extraction surgery. The lens capsule integrity is restored by inserting one or more collagen patches during endocapsular lens extraction surgery to seal the anterior capsulotomy and restore its continuity, which thereby improves the shape and structure of the regenerated lenses. It will be appreciated by those skilled in the art that a variety of collagen patches may be used and that the sealing of the capsulotomy may occur in various regions in connection with various embodiments of the present invention. For example, a collagen patch that is composed of bovine collagen type IV or a 12 hour collagen shield (Chiron Ophthalmics, Emeryville, California, U.S.A.) or a 24 or 72 hour PROSHIELD^{®} Collagen Corneal Shield (Alcon Laboratories, Inc, Fort Worth, TX) may be used in accordance with an embodiment of the present invention. Additionally, a collagen patch may be used to seal any opening in the lens capsule bag, not just the anterior capsulotomy. Furthermore, in an alternate embodiment, injectable collagen may be used as a supplement to or a replacement for the inserted collagen patch to further enhance lens regeneration.

In an additional embodiment, collagen may be used as an internal scaffold for lens fiber cell proliferation and differentiation. A variety of collagen-based products may be used, as for example, 25% or 50% suspensions of purified bovine dermis in saline with 0.3% lidocaine (available under the trade name Zyderm I^{®} and Zyderm II^{®} from INAMED Corporation; Santa Barbara, California), monomolecular bovine collagen suspended in solution at 3.5% and 6.5% concentrations (available under the trade name Resoplast^{®} from Rofil Medical International; Breda, Holland), human collagen preparation comprised predominantly of intact collagen fibers as well as other matrix proteins suspended in a neutral pH buffer (available under the trade name Dermalogen^{®} from Collagenesis Corporation; Beverly, Massachusetts), a cellular human dermal graft processed from tissue bank-derived skin (available under the trade name Alloderm^{®} from LifeCell Corporation; Palo Alto, California), GAG-based compound or polymer; and/or include collagen produced by amnion as described in U.S. Patent Application Publication No. 2004/0048796 (Serial No. 10/397,867).

In those embodiments of the present invention directed to injectable lens materials for treating ocular disease and/or correcting vision impairment, one can use these methods to treat any disease in which enhancing lens regeneration has a beneficial effect on a patient (e.g., ameliorating a disease, lessening the severity of its complications, preventing it from manifesting, preventing it from recurring, merely preventing it from worsening, or a therapeutic effort to effect any of the aforementioned, even if such therapeutic effort is ultimately unsuccessful). Materials of the present invention may be used to treat any diseases which are affected by lens tissue loss or damage, or ocular conditions or impairments which involve a medical procedure comprising the removal or alteration of lens tissue.

The present invention also includes kits. In one embodiment, the kits of the present invention comprise injectable lens materials including, in certain embodiments other materials to assist in the enhancement of lens regeneration. Kits of the present invention can contain one or more of the following in a package or container: (1) one or more injectable lens materials of the present invention; (2) one or more pharmaceutically acceptable adjuvants or excipients; (3) one or more vehicles for administration, such as one or more syringes; (4) one or more tools to use in a surgical procedure; (5) one or more additional bioactive agents for concurrent or sequential administration and/or (6) instructional information. Embodiments in which two or more of components (1) - (6) are found in the same container can also be used.

When a kit is supplied, the different components of the compositions included can be packaged in separate containers and admixed immediately before use. Such packaging of the components separately can permit long-term storage without losing the active components' functions. When more than one composition or active agent is included in a particular kit, the bioactive agents may be (1) packaged separately and admixed separately with appropriate (similar or different) vehicles immediately before use, (2) packaged together and admixed together immediately before use or (3) packaged separately and admixed together immediately before use. If the chosen compounds will remain stable after admixture, however, the admixture need not occur immediately before use but can occur at a time before use, including in one example, minutes, hours, days, months or years before use or in another embodiment at the time of manufacture.

The compositions included in particular kits of the present invention can be supplied in containers of any sort such that the life of the different components are preserved and are not adsorbed or altered by the materials of the container and/or so that other components are not damaged. For example, sealed glass ampules can contain lyophilized agents or variants or derivatives thereof or other bioactive agents, or buffers that have been packaged under a neutral, non-reacting gas, such as, without limitation, nitrogen. Ampules can consist of any suitable material, such as, without limitation, glass, organic polymers, such as, polycarbonate, polystyrene, etc., ceramic, metal or any other material typically employed to hold similar reagents. Other examples of suitable containers include, without limitation, simple bottles that may be fabricated from similar substances as ampules, and envelopes that can comprise foil-lined interiors, such as aluminum or an alloy. Other containers include, without limitation, test tubes, vials, flasks, bottles, syringes, or the like. Containers can have one or more sterile access ports, such as a bottle having a stopper that can be pierced by a hypodermic injection needle. Other containers may have two compartments that are separated by a readily removable membrane that upon removal permits the components to be mixed. Removable membranes may be, without limitation, glass, plastic, rubber, etc.

As stated earlier, kits can also be supplied with instructional materials. Instructions may be printed on paper or other substrate, and/or may be supplied as an electronic-readable medium, such as a floppy disc, CD-ROM, DVD-ROM, Zip disc, videotape, audiotape, flash memory device, etc. Detailed instructions may not be physically associated with the kit; instead, a user may be directed to an internet web site specified by the manufacturer or distributor of the kit, or supplied as electronic mail.

As should be understood, the exact formulation, route of administration, and dosage should generally be determined by the attending physician in view of the patient's condition. Dosage amount and interval can be adjusted individually to provide appropriate levels of nucleic acid molecules which are sufficient to maintain therapeutic effect.

## Claims

1. An injectable lens material comprising a polysiloxane polymer, for use in a method of treating an ocular condition by enhancing regeneration of lens cells in a mammal after endocapsular extraction, the method comprising filling a lens capsule bag of said mammal with the injectable lens material, wherein said injectable lens material has a viscosity for being injected through standard cannula and said polymer has functional groups at terminal ends of said polymer.

2. An injectable lens material according to claim 1, wherein said endocapsular extraction occurs through a capsulorrhexis that is 3 millimeters or less.

3. An injectable lens material according to claim 1, wherein said method further comprises inserting a foldable intraocular lens into said capsule bag of said mammal.

4. An injectable lens material according to claim 1, wherein a contact lens or similar polymeric material in the form of a permeable or semi-permeable disc shaped lens is positioned between the anterior capsule and said injectable lens material or positioned between said injectable lens material and the posterior capsule.

5. An injectable lens material according to claim 1, wherein said method further comprises administering a viscoelastic material to said capsule bag of said mammal.

6. An injectable lens material according to claim 5, wherein said viscoelastic material is selected from the group consisting of hyaluronic acid, cellulosic materials, collagen, and combinations thereof.

7. An injectable lens material according to claim 6, wherein said viscoelastic material is hyaluronic acid and said hyaluronic acid is a crosslinked hyaluronic acid.

8. An injectable lens material according to claim 5, wherein said method further comprises the administration of hyaluronidase.

9. An injectable lens material according to claim 1, wherein said injectable lens material further comprises a photoinitiator and is capable of being photopolymerized into a solid intraocular lens.

10. An injectable lens material according to claim 1, wherein said polysiloxane polymer has a backbone of the general formula: 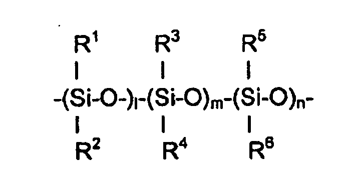 wherein R¹ and R² are independently C₁-C₆ alkyl; R³ is phenyl; R⁴ is phenyl or C₁-C₆ alkyl; R⁵ is CF₃(CH₂)ₓ wherein x is 1-5; R⁶ is C₁-C₆ alkyl or fluoroalkyl; I is in the molar fraction range of 0 to 0.95; m is in the molar fraction range of from greater than 0 to 0.7; and n is in the molar fraction range of from greater than 0 to 0.65.

11. An injectable lens material according to claim 1, wherein said method further comprises inserting at least one collagen patch in said capsule bag of said mammal.

## Patentansprüche

1. Injizierbares Linsenmaterial, umfassend ein Polysiloxanpolymer, zur Verwendung in einem Verfahren zur Behandlung eines Okularzustandes durch Verstärkung der Regeneration von Linsenzellen in einem Säuger nach endokapsularer Extraktion, wobei das Verfahren das Füllen eines Linsenkapselbeutels des Säugers mit dem injizierbaren Linsenmaterial umfasst, wobei das injizierbare Linsenmaterial eine Viskosität aufweist, zum Injizieren durch eine Standardkanüle, und wobei das Polymer funktionelle Gruppen an terminalen Enden des Polymers hat.

2. Injizierbares Linsenmaterial nach Anspruch 1, worin die endokapsulare Extraktion durch eine Kapsulorrhexie auftritt, die 3 mm oder weniger ist.

3. Injizierbares Linsenmaterial nach Anspruch 1, worin das Verfahren weiterhin das Einfügen einer faltbaren Intraokularlinse in den Kapselbeutel des Säugers umfasst.

4. Injizierbares Linsenmaterial nach Anspruch 1, worin eine Kontaktlinse oder ein ähnliches polymeres Material in der Form einer permeablen oder semipermeablen scheibenförmigen Linse zwischen der vorderen Kapsel und dem injizierbaren Linsenmaterial oder dem injizierbaren Linsenmaterial und der hinteren Kapsel positioniert ist.

5. Injizierbares Linsenmaterial nach Anspruch 1, worin das Verfahren weiterhin das Verabreichen eines viskoelastischen Materials an den Kapselbeutel des Säugers umfasst.

6. Injizierbares Linsenmaterial nach Anspruch 5, worin das viskoelastische Material ausgewählt ist aus der Gruppe bestehend aus Hyaluronsäure, Cellulosematerialien, Collagen und Kombinationen davon.

7. Injizierbares Linsenmaterial nach Anspruch 6, worin das viskoelastische Material Hyaluronsäure ist und die Hyaluronsäure eine vernetzte Hyaluronsäure ist.

8. Injizierbares Linsenmaterial nach Anspruch 5, worin das Verfahren weiterhin die Verabreichung von Hyaluronidase umfasst.

9. Injizierbares Linsenmaterial nach Anspruch 1, worin das injizierbare Linsenmaterial weiterhin einen Photoinitiator umfasst und in der Lage ist, in eine feste Intraokularlinse fotopolymerisiert zu werden.

10. Injizierbares Linsenmaterial nach Anspruch 1, worin das Polysiloxanpolymer ein Rückgrat mit der folgenden Formel hat: 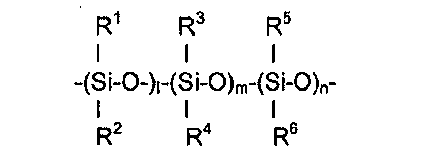 worin R¹ und R² unabhängig C₁-C₆-Alkyl sind, R³ Phenyl ist, R⁴ Phenyl oder C₁-C₆-Alkyl ist, R⁵ CF₃(CH₂)ₓ ist, worin x 1 bis 5 ist, R⁶ C₁-C₆-Alkyl oder -Fluoralkyl ist, 1 ein Molarfraktionsbereich von 0 bis 0,95 ist, m ein Molarkfraktionsbereich von mehr als 0 bis 0,7 ist und n ein Molarfraktionsbereich von mehr als 0 bis 0,65 ist.

11. Injizierbares Linsenmaterial nach Anspruch 1, worin das Verfahren weiterhin das Einfügen von zumindest einem Collagenpatch in den Kapselbeutel des Säugers umfasst.

## Revendications

1. Matière de lentille injectable comprenant un polymère de polysiloxane, destinée à une utilisation dans un procédé de traitement d'un état oculaire en améliorant la régénération de cellules de cristallin chez un mammifère après extraction endocapsulaire, le procédé comprenant le remplissage d'un sac capsulaire de cristallin dudit mammifère avec la matière de lentille injectable, dans lequel ladite matière de lentille injectable a une viscosité pour être injectée par l'intermédiaire d'une canule standard et ledit polymère a des groupes fonctionnels à des extrémités terminales dudit polymère.

2. Matière de lentille injectable selon la revendication 1, dans laquelle ladite extraction endocapsulaire se fait par l'intermédiaire d'une capsulorhexis qui est de 3 millimètres ou moins.

3. Matière de lentille injectable selon la revendication 1, dans laquelle ledit procédé comprend en outre l'insertion d'une lentille intraoculaire pliable dans ledit sac capsulaire dudit mammifère.

4. Matière de lentille injectable selon la revendication 1, dans laquelle une lentille de contact ou une matière polymérique similaire sous la forme d'une lentille en forme de disque perméable ou semi-perméable est positionnée entre la capsule antérieure et ladite matière de lentille injectable ou positionnée entre ladite matière de lentille injectable et la capsule postérieure.

5. Matière de lentille injectable selon la revendication 1, dans laquelle ledit procédé comprend en outre l'administration d'une matière viscoélastique audit sac capsulaire dudit mammifère.

6. Matière de lentille injectable selon la revendication 5, dans laquelle ladite matière viscoélastique est choisie parmi le groupe consistant en l'acide hyaluronique, des matières cellulosiques, le collagène, et des combinaisons de ceux-ci.

7. Matière de lentille injectable selon la revendication 6, dans laquelle ladite matière viscoélastique est l'acide hyaluronique et ledit acide hyaluronique est un acide hyaluronique réticulé.

8. Matière de lentille injectable selon la revendication 5, dans laquelle ledit procédé comprend en outre l'administration de hyaluronidase.

9. Matière de lentille injectable selon la revendication 1, dans laquelle ladite matière de lentille injectable comprend en outre un photoinitiateur et est apte à être photopolymérisée en une lentille intraoculaire solide.

10. Matière de lentille injectable selon la revendication 1, dans laquelle ledit polymère de polysiloxane a un squelette ayant la formule générale: 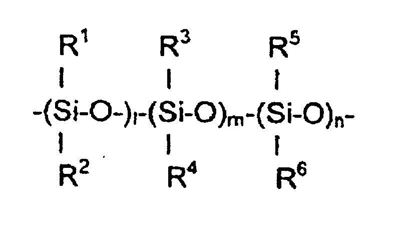 dans laquelle R¹ et R² sont indépendamment un alkyle en C₁-C₆ ; R³ est un phényle; R⁴ est un phényle ou un alkyle en C₁-C₆ ; R⁵ est CF₃(CH₂)ₓ où x est 1 à 5 ; R⁶ est un alkyle en C₁-C₆ ou un fluoroalkyle ; 1 est dans la plage de fraction molaire de 0 à 0,95 ; m est dans la plage de fraction molaire de plus de 0 à 0,7 ; et n est dans la plage de fraction molaire de plus de 0 à 0,65.

11. Matière de lentille injectable selon la revendication 1, dans laquelle ledit procédé comprend en outre l'insertion d'au moins une pièce de collagène dans ledit sac capsulaire dudit mammifère.
